(19) Europäisches Patentamt
European Patent Office
Office européen des brevets

(11) EP 1 818 060 A1

(12) EUROPEAN PATENT APPLICATION

(43) Date of publication:
**15.08.2007 Bulletin 2007/33**

(51) Int Cl.:
***A61K 36/74*** *(2006.01)* ***A61P 17/00*** *(2006.01)*

(21) Application number: **05111134.2**

(22) Date of filing: **23.11.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **ARTSANA S.p.A.**
**22070 Grandate (Como) (IT)**

(72) Inventors:
- **Aquino, Rita**
  **83100 Avellino (IT)**
- **Mencherini, Teresa**
  **84081 Baronissi (SA) (IT)**

(74) Representative: **Mancini, Vincenzo**
**Giambrocono & C. S.p.A.**
**Via Rosolino Pilo 19/B**
**20129 Milano (IT)**

# (54) Cosmetic composition comprising Fadogia ancylantha extracts

(57) The present invention relates to a topical cosmetic and/or dermopharmaceutical composition comprising *Fadogia ancylantha* extracts and/or ingredients and the use thereof.

The invention relates to the field of cosmetics and, in particular, to the use of such composition, an extract or a cosmetically and/or dermopharmaceutically active substance of *Fadogia ancylantha* for the cosmetic and/or dermopharmaceutical treatment of the human body.

EP 1 818 060 A1

## Description

The present invention relates to a topical cosmetic and/or dermopharmaceutical composition comprising *Fadogia ancylantha* extracts and/or ingredients and the use thereof.

The invention relates to the field of cosmetics and, in particular, to the use of such composition, an extract or a cosmetically and/or dermopharmaceutically active substance of *Fadogia ancylantha* for the cosmetic and/or dermopharmaceutical treatment of the human body.

A key reason for the ageing of skin is the loss of water from the upper layers of the epidermis and the wrinkling associated therewith. Further, environmental factors concur decidedly to accelerate and anticipate skin ageing, this making evident the importance of cosmetic prevention which can lead to remarkable improvements of skin tone and to the reduction of micro-wrinkles, bringing to a general effective intervention on skin degenerative phenomena.

Further, an increase of skin sensibility and of the intolerance towards detergents, cosmetics, pollution, cold, wind, exposition to sun rays, are among the reasons why cosmetic and dermopharmaceutical products must be specifically elaborated and subjected to severe test as long as skin tolerability and allergic responses are concerned; an increase of allergies and dermatitises, in particular, of skin reactions to aluminium salts, which are widely used in the cosmetic and dermopharmaceutical fields and also an increased attention of the consumer towards chemical components, which urges for natural active principles, especially deriving from plants and/or the extracts thereof, have come to the attention of the public.

Searching for alternatives in the cosmetic and dermopharmaceutical fields, both functionally effective and well tolerated by skin, is therefore of great importance.

Accordingly, one of the ways cosmetic chemists and experts seek to counter these phenomena is to provide active substances, of natural origin, which counteract environmental stress and dehydration and/or which have a protective function so that the cells are fortified in their ongoing struggle against environmental poisons.

It has surprisingly been found that the extracts and/or the cosmetically and/or dermopharmaceutically active substances of *Fadogia ancylantha* solve the problems above illustrated in an excellent fashion. The extracts may be used as such although individual constituents may also be isolated from them and then mixed in a different composition according to requirements.

*Fadogia ancylantha (Cissus ibuensis,* common name: Makoni or Tulimara) is a wild perennial bush of the *Rubiaceae* family, which mainly grows in Zimbabwe.

The leaves of this bush have been used, *inter alia,* to boost the immune system, tone muscles, strengthen bones, as a poison antidote, as an aphrodisiac in the local medicine; the antispasmodial activity has further been reported by Sanon S. et al. in "Ethnobotanical survey and in vitro antispasmodial activity of plants used in traditional medicine in Burkina Faso", J. of Ethnopharmacology 2003, 86 (2-3), 143-7.

Mostly, the leaves of *Fadogia ancylantha* are fermented and then dried so to produce Makoni tea which is commercialised by SAFIRE (Southern Alliance for Indigenous Resources), a non-governmental organization.

According to a first aspect, the invention relates to a topical cosmetic and/or dermopharmaceutical composition comprising at least one effective amount of a plant ingredient or plant extract from *Fadogia Ancylantha* and/or parts thereof, with the proviso that the quantitative data add up to 100% by weight with a) at least one cosmetically and/or dermopharmaceutically acceptable preservative-antioxidant, and b) water or at least one cosmetically and/or dermopharmaceutically acceptable excipient-emulsifier-liophilic substance, and optionally c) at least one cosmetically and/or dermopharmaceutically acceptable auxiliary.

In the present specification, the expression "plant extract" means generally the residual deriving from the extraction of *Fadogia ancylantha* or parts of this plant whereas "plant ingredient" means generally at least one of the substances contained in the plant or in at least a part thereof or in a plant extract.

It is preferred that the at least one plant ingredient or plant extract is present in an amount from about 0.1 to about 5.0%, in particular from about 0.3 to about 3.0%, most preferably from about 0.5 to about 1.0% by weight of the total composition.

The expression "cosmetically and/or dermopharmaceutically acceptable preservative-antioxidant" is herein used to generally mean any substance showing such functions known to the skilled man and, in particular selected, for instance, from the group consisting of benzoic acid and the salts and esters thereof, benzyl alcohol, BHA, cetrimonium chloride, diazolidinyl urea, DMDM hydantoin, EDTA and the salts thereof, imidazolidinyl urea, methylchloroisothiazolinone, methylisothiazolinone, paraben salts and esters, phenoxyethanol, polyquaternium, quaternium, sodium dehydroacetate, triclosan, sorbic acid.

The expression "cosmetically and/or dermopharmaceutically acceptable auxiliary" is herein used to generally mean any substance showing a vehiculating ability known to the skilled man and, in particular one or more ingredients selected, for instance, from the group consisting of additives-formulation auxiliaries-solvents; surfactants-solubilizers; natural substances; polymers-rheological modifiers; silicone derivatives; pigments-inorganic additives; humectants; functional substances; functional additives.

It is also preferred that the plant extract and/or ingredient comprise at least one of the following compounds:

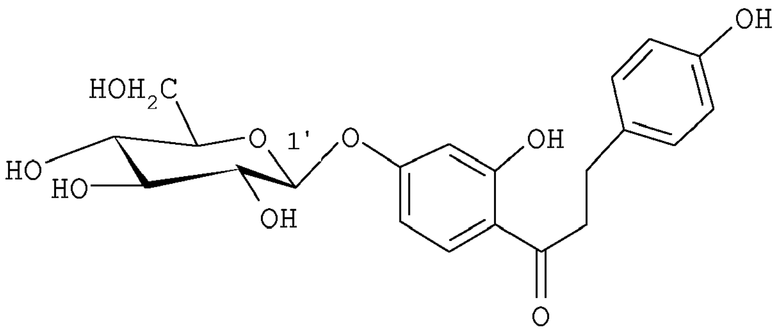

(1)

4'-β-O-D-glucopyranosyl-4,2',4'-trihydroxydihydrochalcone;

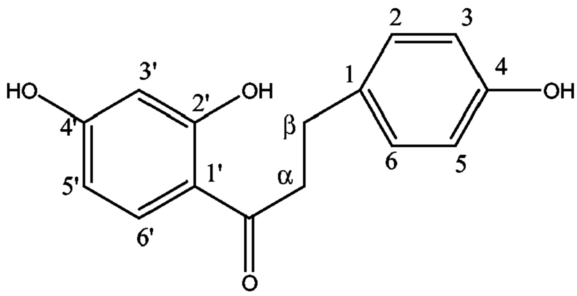

(2)

4,2',4'-trihydroxydihydrochalcone;

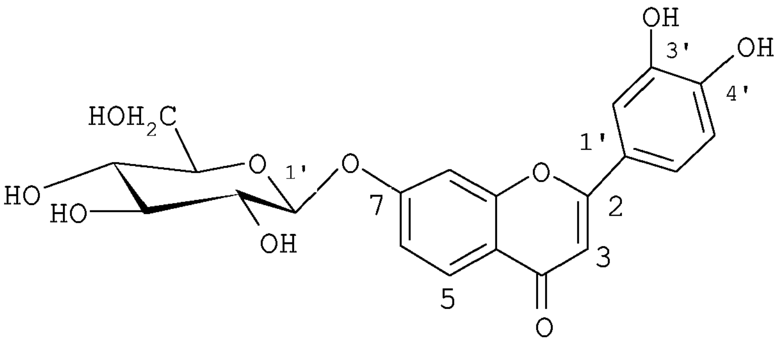

(3)

7-O-β-D-glucopyranosyl-7,3',4'-trihydroxyflavone;

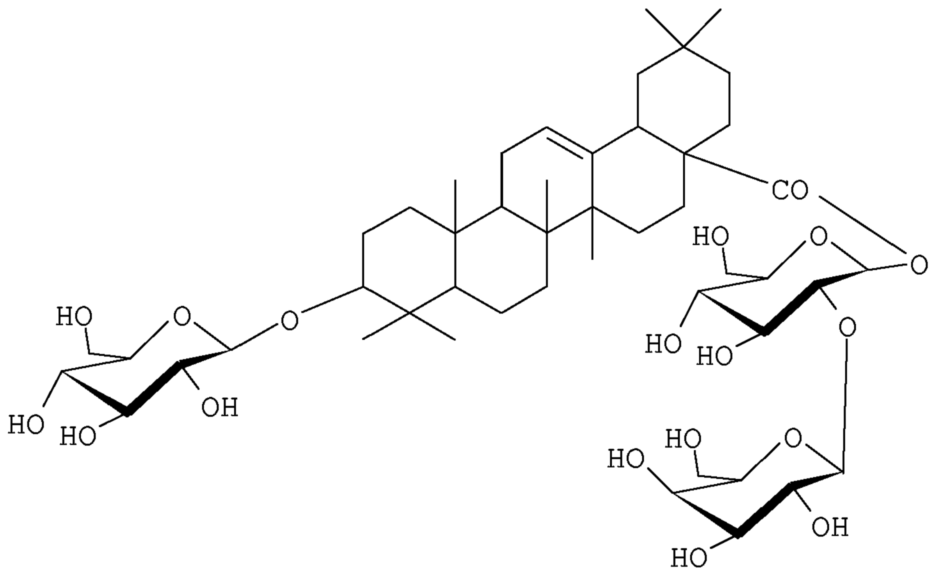

(4)

3-O-β-D-glucopyranosyl-olean-12-en-28-O-[β-D-glucopyranosyl (1→2) galactopyranoside].

The extract comprised in the composition of the invention is obtainable by

a) extracting the plant *Fadogia Ancylantha* or parts of this plant with a solvent selected from the group consisting of water, a $C_1$-$C_4$ alcohol and mixtures thereof so that a solution of the extract in the solvent is obtained, and
b) removing the solvent from this solution, so that the extract is obtained.

As above noted, the extract comprised in the composition of the invention is obtainable, for instance and preferably, by aqueous, alcoholic or aqueous/alcoholic extraction (methanol, ethanol and aqueous solutions of these two alcohols are particularly preferred) of the plants or parts thereof, although other conventional solvents and extraction processes, such as maceration, remaceration, digestion, agitation maceration, vortex extraction, ultrasonic extraction, countercurrent extraction, percolation, repercolation, evacolation (extraction under reduced pressure) and solid/liquid extraction under continuous reflux in a Soxhlet extractor, which are well known to the skilled man, may all be used in principle.

Fresh plants or parts thereof are suitable as the starting material although dried plants and/or plant parts, most preferably the leaves, which may be mechanically size-reduced before extraction are normally used. Any size reduction methods known to the skilled man, such as freeze grinding for example, may be used.

Suitable solvents for the extraction process are organic solvents, water (preferably hot water with a temperature above 20°C and, in particular, 20 to 30°C) or mixtures of organic solvents and water, more particularly low molecular weight alcohols with water contents. The extraction process is generally carried out at 25°C. The extraction times are selected by the skilled man in dependence upon the starting material, the extraction process, the extraction temperature and the ratio of solvent to raw material, etc. After the extraction process, the crude extracts obtained may optionally be subjected to other typical steps, such as for example purification, concentration and/or decoloration. If desired, the extracts thus prepared may be subjected, for example, to the selective removal of individual unwanted ingredients. The extraction process may be carried out to any degree, but is usually continued to exhaustion, finally removing the solvent completely by drying, more particularly by spray drying or freeze drying. The extraction conditions and the yields of the final extracts may be selected by the skilled man according to the desired application. These extracts show generally active substance contents (i.e. plant ingredients) of about 40.0 to 50.0% by weight.

The extracts may also be used as starting materials for the preparation of the pure active substances where they cannot be produced more simply and inexpensively by the synthetic route. Instead of the extracts, the active substances (i.e. plant ingredients) present in the survival fractions may also be used individually or in the form of mixtures. They may be products obtained by purifying the extracts or by synthetic routes. The products obtainable from the extracts according to the invention by purification are particularly preferred.

The composition of the invention can be realized in substantially any of the product form known to the skilled man; for example, foam baths, shower baths, creams, gels, lotions, alcoholic and aqueous/alcoholic solutions, emulsions, wax/fat compounds, stick preparations, powders or ointments. These preparations may also contain mild surfactants, oil components, emulsifiers, pearlizing waxes, consistency factors, thickeners, superfatting agents, stabilizers, polymers,

silicone compounds, fats, waxes, lecithins, phospholipids, biogenic agents, UV protection factors, antioxidants, deodorants, antiperspirants, film formers, swelling agents, solubilizers, preservatives, perfume oils, and the like as further auxiliaries and additives.

In particular, the composition is in a product form selected from the group consisting of aerosol, cream, emulsion, solid, liquid, dispersion, foam, gel, lotion, micro-system, modulated lipophilic systems, mousse, nano-system, ointment, powder, patch, pomade, serum, solution, pump spray, stick, wet wipes, towelette, and combinations thereof.

Particularly preferred are oil/water, water/oil, water/oil/water, oil/water/oil, gel, liquid crystal, silicone, cold emulsions, microemulsions and creams, modulated lipophilic systems (oils, sera and gels), patch, micro- and nano- release systems.

Following his knowledge, the skilled man can easily formulate the composition of the invention so that it preferably comprises at least one cosmetically and/or dermopharmaceutically acceptable auxiliary including, in particular, one or more ingredients selected from the group consisting of additives-formulation auxiliaries-solvents; surfactants-solubilizers; natural substances; polymers-rheological modifiers; silicone derivatives; pigments-inorganic additives; humectants; functional substances; functional additives.

As already noted above, the quantitative data of the composition of the invention add up to 100% by weight with a) at least one cosmetically and/or dermopharmaceutically acceptable preservative-antioxidant, and b) water or at least one cosmetically and/or dermopharmaceutically acceptable excipient-emulsifier-liophilic substance, and optionally c) at least one cosmetically and/or dermopharmaceutically acceptable auxiliary.

Suitable preservatives/antioxidants can be selected, for instance, from the group consisting of benzoic acid and the salts and esters thereof, benzyl alcohol, BHA, cetrimonium chloride, diazolidinyl urea, DMDM hydantoin, EDTA and the salts thereof, imidazolidinyl urea, methylchloroisothiazolinone, methylisothiazolinone, paraben salts and esters, phenoxyethanol, polyquaternium, quaternium, sodium dehydroacetate, triclosan, sorbic acid; they may be present in the preparations in quantities of preferably about 0.01 to 3.0% by weight, more preferably 0.05 to 2.0% by weight of the total composition.

Suitable excipients/emulsifiers/liophilic substances can be selected, for instance, from the group consisting of acetylated lanolin alcohol, beheneth-25, C 12-15 alkyl benzoate, $C_{10}$-$C_{18}$ triglycerides, $C_{12}$-$C_{15}$ pareth-7, $C_{18}$-$C_{70}$ isoparaffin, caprylic/capric triglyceride, ceteareth- 2 and 20, cetearyl alcohol, cetearyl isononanoate, cetearyl palmitate, coco-caprylate/caprate, cocoglycerides, dibutyl adipate, dicaprylyl carbonate, dicaprylyl ether, dimyristyl tartrate, dipropylene glycol, distearyl ether, ethylhexyl palmitate, ethylhexyl stearate, glyceryl oleate, glyceryl stearate, glycol distearate, hydrogenated polyisobutene, isopropyl myristate, isostearyl avocadate, lanolin and the derivatives thereof, laureth-2, lecithin, linseed acid, myristyl myristate, octyl palmitate, octyldodecanol, paraffinum liquidum, peg-2 hydrogenated castor oil, peg-5 glyceryl stearate, peg-7 glyceryl cocoate, pentaerythrityl tetraisostearate, petrolatum, polyethylene glycols (PEG), polyglyceryl-2 dipolyhydroxystearate, polyglyceryl-3 diisostearate, polyglyceryl-6 polyhydroxystearate, sodium malate-, sorbitan sesquioleate, sorbitan isostearate, squalane, steareth- 2 and 21, stearic acid, sucrose polystearate, trideceth-6; they may be present in the preparations in quantities of preferably about 0.1 to 99.89% by weight, more preferably 1.0 to 99.65% by weight of the total composition.

Suitable additives/formulation auxiliaries/solvents can be, for instance, selected from the group consisting of water, $C_1$-$C_4$ alcohols, citric acid, dextran sulphate, ethoxydiglycol, lactic acid and the salts thereof, sodium chloride, triethanolamine; they may be present in the preparations in quantities of preferably about 0.01 to 95.0% by weight, more preferably 0.1 to 90.0% by weight of the total composition.

Suitable surfactants/solubilizers can be selected, for instance, from the group consisting of betaine, cocamide DEA, cocamide MEA, cocamidopropyl betaine, coco glucoside, decyl glucoside, PEG-40 hydrogenated castor oil, PEG-7 glyceryl cocoate, polyquaternium-7, PPG-25 laureth-25, PPG-26 buteth 26, Sodium laureth sulfate, sodium myreth sulfate; they may be present in the preparations in quantities of preferably about 0.5 to 50.0% by weight, more preferably 2.0 to 50.0% by weight of the total composition.

Suitable natural substances can be selected, for instance, from the group consisting of *Aesculus Hippocastanum,* algae, *Aloe Barbadensis,* apricot kernel oil, *Avena Sativa, Bacopa Monniera, Borago Officinalis, Butyris Lac, Butyrospermum Parkii, Calendula Officinalis, Centaurea Cyanus, Centella Asiatica,* Alba wax, Microcrystalline wax, *Chamomilla Recutita, Cocos Lucifera, Corylus Avelanna, Crataegus Monogina, Epilobium Angustifolium, Eupleurum Falcatum, Ginkgo Biloba, Glicine Soja, Hamamelis Virginiana, Helianthus annuus, Helychrysum Arenarium, Helychrysum Italicum, Luffa Cilindrica, Macrocystis Pyrifera, Mal va Syl vestris, Margaritifera Margaritifera, Oenothera Biennis, Olea Europea, Oryza Sativa, Ozokerite, Passiflora Incarnata, Pelvetia Canaliculata, Persea Gratissima, Prunus Armeniaca, Prunus Dulcis, Rosmarinus Officinalis, Ruscus Aculeatus, Simmondia Chinensis, Solanum Lycopersicum, Teobroma Cacao, Triticum Vulgare, Zea Mays, Melissa Officinalis;* they may be present in the preparations in quantities of preferably about 0.01 to 95.0% by weight, more preferably 0.1 to 90.0% by weight of the total composition.

Suitable polymers/rheological modifiers can be selected, for instance, from the group consisting of acacia Senegal gum, acrylates/$C_{10}$- $C_{30}$ alkyl acrylate crosspolymer, biosaccharide gum-4, carbomer, carboxymethyl chitosan, hyaluronic acid, hydrogenated butylene ethylene styrene copolymer, hydrogenated dimer diilinoleyl/dimethylcarbonate

copolymer, hydrogenated ethylene propylene styrene copolymer, hydroxyethylcellulose, PEG-45/dodecyl glycol copolymer, propylene glycol arginate, sodium styrene/acrylates/divinylbenzene copolymer, sodium carboxymethyl betaglucan, sodium polyacrylate, tricontanyl PVP, xanthan gum; they may be present in the preparations in quantities of preferably about 0.1 to 10.0% by weight, more preferably_0.5 to 5.0% by weight of the total composition.

Suitable silicone derivatives can be selected, for instance, from the group consisting of bis-PEG-18 methyl ether dimethyl silane, cetyl dimethicone copolyol, cyclosiloxanes, dimethicone, dimethicone copolyol, dimethicone crosspolymer, polysiloxanes, silica dimethyl silylate, siloxanetriol arginate, triethoxycaprylylsilane; they may be present in the preparations in quantities of preferably about 0.1 to 90.0% by weight, more preferably 1.0 to 90.0% by weight of the total composition.

Suitable pigments/inorganic additives can be selected, for instance, from the group consisting of alumina, aluminium stearate, bentonite, iron oxides, magnesium aluminium silicate, magnesium sulphate, mica, silica, talc, titanium dioxide, zinc oxide; they may be present in the preparations in quantities of preferably about 0.01 to 40.0% by weight, more preferably 0.1 to 30.0% by weight of the total composition.

Suitable humectants can be selected, for instance, from the group consisting of butylene glycol, dipropylene glycol, glycerin, glycols, polyglycols; they may be present in the preparations in quantities of normally about 0.1 to 60.0% by weight, more preferably 1.0 to 20.0% by weight of the total composition.

Suitable functional substances can be selected, for instance, from the group consisting of 4-methyl benzylidene camphor, acetyl dipeptide-1 cetyl esters, allantoin, aluminium chloride, aluminium chlorohydrate, aluminium starch octenylsuccinate, aminoacids and the derivatives thereof, ammonium glycyrrhizate, benzophenone-3, benzophenone-4, benzyl salycilate, bisabolol, butyl methoxydibenzoyl methane, caffeine, ceramide 2, diethylamino hydroxybenzoyl hexyl benzoate, EFA, escin, ethylhexyl methoxycinnamate, ethylhexyl triazone, glutathione, glycolic acid, hesperidin methyl chalcone, hydrolyzed yeast protein, lauryl aminopropylglycine, menthyl lactate, octyl triazone, Peg-10 rapeseed sterol, peptides-, potassium caproyl tyrosine, ruscogenin, *Saccharomyces* lysate extract, serum albumin, trehalose, urea, vitamins and the derivatives thereof; they may be present in the preparations in quantities of normally about 0.01_ to 30.0% by weight, preferably 0.1 to 20.0% by weight of the total composition.

Further, a number of functional additives can be comprised in the above defined composition, in addition to the plant extract and/or ingredient of *Fadogia ancylantha,* in order to get an increased and/or additional activity and, therefore, a more effective result against the treated condition; they may be present in the preparations in quantities of normally about 0.1 to 25.0% by weight, preferably 0.5 to 20.0% by weight of the total composition.

Among the preferred active principles, the following can be mentioned by way of example.

Substances having an activity against free radicals such as, for instance, vitamins, tocopheryl acetate, magnesium ascorbyl phosphate, retinyl acetate, *Prunus Armeniaca, Solanum Lycopersicum, Rosmarinus Officinalis, Theobroma Cacao.*

Substances having an activity increasing skin firming such as, for instance, palmitoyl pentapeptide-3, acetyl dipeptide-1 cetyl esters, Zea Mays kernel Extract, Faex, *Macrocystis Pyrifera,* methylsilanol hydroxyproline aspartate, *Centella Asiatica,* carnitine, bioflavonoids, caffeine, algae, soy isoflavones.

Substances having an activity increasing the skin barrier function such as, for instance, biosaccharide gum-4, trehalose, hydrolyzed glycosaminoglycans, carboxymethyl chitosan, jaluronic acid, ceramide 3, ceramide 6-II, ceramide 1, phytosphingosine, cholesterol, sodium lauroyl lactylate, hydrolyzed *Adansonia Digitata* extract, octydodecanol, hydrogenated coco-glycerides, *Helianthus Annuus* seed extract.

Substances stimulating cellular renewal such as, for instance, retinol, lauroyl silk amino acids, sodium malate, glycolic acid, *Dulaniella Salina,* spirulin, yeast extract, *Saccharomyces* lysate extract, *Artemia Salina.*

Substances which act on bioadhesion such as, for instance, mannitol and yeast extract.

Substances having an activity on melanogenesis either of the inhibition or promoting type such as, for instance, oleoyl tyrosine, oleic acid; tyrosine; erythrulose; potassium caproyl tyrosine; willowbark extract; *Citrus Dulcis, Oriza Sativa, Capparis Spinosa, Olea Europaea;* magnesium ascorbyl phosphate. Substances having a lifting-like effect such as, for instance, *Quillaja Saponaria, Kigelia Africana, Rosmarinus Officinalis;* serum albumin, hyaluronic acid, dextran sulfate, *Prunus Amygdalus Dulcis, Argania Spinosa* kernel extract, sodium cocoyl gluatamate. Zea Mays kernel extract, pentaerythrityl tetraisostearate, silica dimethyl silylate, hydrolyzed wheat protein, lactobionic acid, oyster shell powder, AHA, BHA, PHA, AKA.

Substances having a filling-like effect on face and eyes such as, for instance,: Zea Mays kernel extract and *Pelvetia Canaliculata.*

Substances having an antiwrinkle effect such as, for instance, peptides.

Phyto-extracts such as, for instance, *Melissa Officinalis, Panax Gingseng, Elichrisium Italicum.*

Substances having a deodorizing antimicrobial activity such as, for instance, aluminium salts, triclosan, triethyl citrate, farnesol, *Melaleuca Alternifolia, Citrus Dulcis.*

Substances having a decongestant activity such as, for instance, bisabolol, glycirrhetic acid, *Avena Sativa* extract.

According to another aspect, the present invention also relates to a compound having the following structural formula:

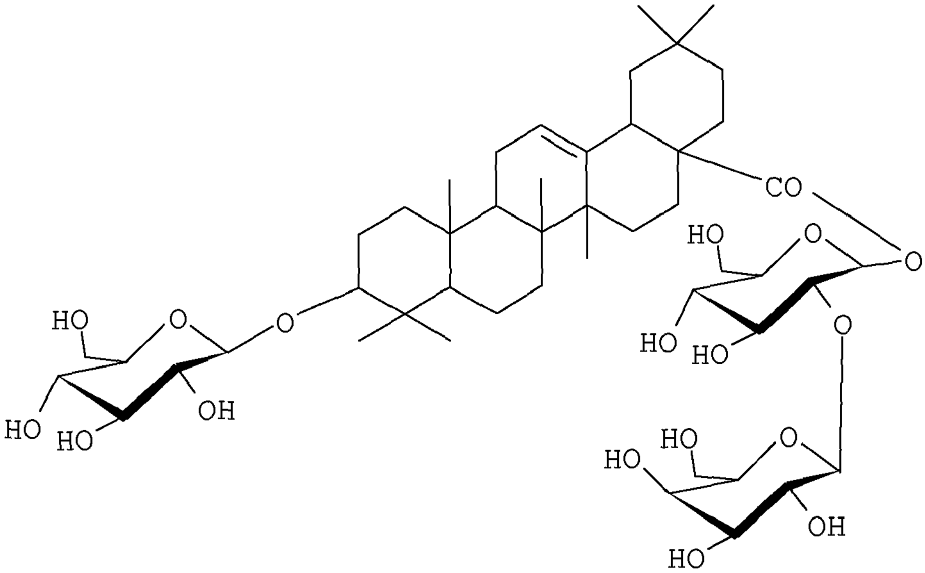

(4)

3-O-β-D-glucopyranosyl-olean-12-en-28-O-[β-D-glucopyranosyl (1→2) galactopyranoside].

The compound of formula (4) above is obtainable by

a) extracting the plant *Fadogia Ancylantha* or parts of this plant with a solvent selected from the group consisting of water, a $C_1$-$C_4$ alcohol and mixtures thereof so that a solution of the extract in the solvent is obtained;
b) removing the solvent from this solution, so that the extract is obtained;
c) freeze-drying and drying the extract;
d) separating the desired compound from the dry freeze-dried extract, for instance by gel filtration chromatography.

The present invention also relates to the use of an extract and/or ingredient of *Fadogia ancylantha* and/or of the composition and/or the compound, as above defined, for the cosmetic and/or dermopharmaceutical treatment of the human body.

In particular, the cosmetic and/or dermopharmaceutical treatment comprises simultaneously an anti-ageing, anti-wrinkle, anti-bacterial, antioxidant and anti-free-radicals effect on aged, photo-aged, stressed or tired skin.

The present invention further concerns a method of improving at least one sign of ageing in skin, comprising topically applying to the skin the composition or compound, as above defined, in a cosmetically or dermopharmaceutically effective amount sufficient to improve at least one sign of ageing in skin. In particular, the at least one sign of ageing is selected from the group consisting of lines, fine lines, wrinkles, crow's feet, dark eye circles, blemishes, age spots, and stretch marks and results from at least one of free radical damage, environmental agents, pollutants, diet, chronological ageing, premature ageing, hormonal ageing, and photo-ageing.

The invention finally relates also to the use of a plant ingredient or plant extract from *Fadogia ancylantha* as active agent having simultaneously protective and repairing properties, for the preparation of a composition, as above defined, for the cosmetic and/or dermopharmaceutical treatment of the human body.

It has been found that the extracts and/or the cosmetically and/or dermopharmaceutically active substances of *Fadogia ancylantha* comprised in the composition of the invention are advantageously suitable for preventing, combatting and/or repairing skin ageing, including photo-ageing and exert both antibacterial and antifungine activity as well.

The composition of the invention can positively affect skin hydration, tone and elasticity and helps opposing to adverse environmental factors and is also well tolerated by skin.

The constant and correct application of the composition of the invention stimulates also the ability of the skin to defend itself and regulate skin homeostasis also inducing the reduction of visible ageing signs of the skin.

The following examples illustrate the invention without limiting it. Unless adversely noted, any percentage is expressed by weight with respect to the total weight of the composition.

Example 1

The leaves (307.89 g) of *Fadogia ancylantha* dried and chopped were extracted, at room temperature, for three times using 3 liters of each of the following solvents, having an increasing polarity: n-hexane (extract C); chloroform (extract B); methanol (extract A).

The leaves were left to macerate in the solvent for four days filtering off the extracts and repeating for three times the maceration in the same conditions.

The extracts resulting from the various macerations were separately dried, under reduced pressure, by a rotating evaporator, to remove the solvent under vacuum and then suspended in water and subsequently subjected to freeze-drying to eliminate any trace of the solvents used for the extractions.

The amounts of the dry freeze-dried extracts thus obtained are illustrated in the following Table

| Extract | Solvent | Amount | Yield % |
|---|---|---|---|
| C | n-hexane | 1.2-1.5g | 0.4-0.5 |
| B | chloroform | 5.5-5.8g | 1.8-2.0 |
| A | methanol | 148.0-148.5g | 47.0-48.0 |

Example 2

In order to isolate the cosmetic and dermopharmaceutically active substances comprised in the dry extract A of the Example 1, 2.8 g of such extract were subjected to gel filtration chromatography (molecular exclusion chromatography), thus obtaining different molecular fractions showing a homogeneous molecular weight.

A Sephadex LH-20 column (Pharmacia) and methanol, as eluent, were used, collecting 5-6 ml fractions which were controlled by thin layer chromatography (TLC) on silica gel (n-butane:acetic acid:water - 60:15:25; chloroform: methanol:water - 80:18:2).

The fractions showing a similar $R_f$ were reunited, selecting the bioactive fractions corresponding to:

| | | | |
|---|---|---|---|
| fractions 38-40 | extract (E') | 100-104 | mg; |
| fractions 78-84 | extract (L') | 380-400 | mg; |
| fractions 85-88 | extract (M') | 49-51 | mg; |
| fractions 92-105 | extract (O') | 190-205 | mg. |

The 'marker' molecules were isolated from the above fractions by RP-HPLC [chromatograph: Waters; pump: Waters 590; injector U6K; refraction index detector: R401; column: $C_{18}$ $\mu$-Bondapack (30 cm x 7.8 mm); flow: 2 ml/min; isocratic method].

The compound 4'-β-O-D-glucopyranosyl-4,2',4'-trihydroxydihydrochalcone, having the above illustrated formula (1), was isolated from fractions L' and M' [Retention time (Rt): 28 min; 156.0 mg and Rt: 30 min; 13,9 mg, respectively] using methanol:water-4:6 as eluent.

The compound 4,2',4'-trihydroxydihydrochalcone, having the above illustrated formula (2) (Rt 68 min; 33.6 mg) and 7-O-β-D-glucopyranosyl-7,3',4'-trihydroxyflavone, having the above illustrated formula (3) (Rt 18 min; 24.1 mg), were isolated from fraction O' using, in both cases, methanol:water 4:6 as eluent.

The compound 3-O-β-D-glucopyranosyl-olean-12-en-28-O-[β-D-glucopyranosyl (1→2) galactopyranoside] having the above illustrated formula (4) (Rt above 60 min; 6.37 mg), was isolated from fraction E' using methanol:water 3:7 as eluent.

The molecular structures of compounds 1-4 were determined by NMR [spectrometer: Bruker DRX 600, equipped with UXNMR software, working at 599.2 MHz for $^1H$ and 150.9 MHz for $^{13}C$] and MS [Electrospray-ionization mass spectrometry-ESIMS, Finnigan LC-Q Deca ion trap instrument (Thermoquest) equipped with Xcalibur software].

The peaks of the NMR spectra [$^{13}C$-NMR and $^1H$-NMR ($CD_3OD$, 600 MHz)] of compounds (1)-(4) are illustrated in the following tables.

| Carbon | Compound (2) | | Aglycone of the compound (1) | | |
|---|---|---|---|---|---|
| | $\delta_C$ | $\delta_H$ | $\delta_C$ | $\delta_H$ | |
| 1 | 133.2 | - | 134.6 | - | C |

(continued)

| Carbon | Compound (2) | | Aglycone of the compound (1) | | |
|---|---|---|---|---|---|
| | $\delta_C$ | $\delta_H$ | $\delta_C$ | $\delta_H$ | |
| 2 | 130.7 | 7.07 | 131.0 | 7.07 | CH |
| 3 | 116.2 | 6.72 | 117.7 | 6.71 | CH |
| 4 | 156.6 | - | 158.2 | - | C |
| 5 | 116.2 | 6.72 | 117.7 | 6.71 | CH |
| 6 | 130.7 | 7.07 | 131.0 | 7.07 | CH |
| α | 40.9 | 3.17 | 42.7 | 3.14 | $CH_2$ |
| β | 31.0 | 2.92 | 32.3 | 2.13 | $CH_2$ |
| CO | 205.5 | - | 207.7 | - | CO |
| 1' | 113.8 | - | 117.5 | - | C |
| 2' | 166.0 | - | 166.6 | - | C |
| 3' | 103.7 | 6.26 | 106.6 | 6.60 | CH |
| 4' | 166.8 | - | 166.5 | - | C |
| 5' | 109.3 | 6.35 | 110.9 | 6.65 | CH |
| 6' | 133.7 | 7.7 | 134.9 | 7.81 | CH |

Sugar of the compound (1)

| position | $\delta_C$ | $\delta_H$ |
|---|---|---|
| 1' | 102.8 | 5.16 |
| 2' | 76.2 | 3.40 |
| 3' | 79.4 | 3.50 |
| 4' | 72.7 | 3.37 |
| 5' | 78.8 | 3.50 |
| 6' | 63.9 | 3.72, 3.92 |

Aglycone of the compound (3)

| Carbon | $\delta_C$ | | $\delta_H$ | Cross peaks ($\delta_C$) in the HMBC spectrum |
|---|---|---|---|---|
| 2 | 147.4 | C | - | |
| 3 | 115.5 | CH | 6.78 | 184.6 (C-4), 147.4 (C-2), 126.7 (C-6), 117.7 (C-2') |
| 4 | 184.6 | CO | - | |
| 5 | 126.4 | CH | 7.72 | 169.1 (C-9), 166.6 (C-7) |
| 6 | 114.8 | CH | 7.00 | 117.2 (C-10), 100.7 (C-8) |
| 7 | 166.6 | C | - | 5.15 (Glucose-1') |
| 8 | 100.7 | CH | 7.12 | 169.1 (C-9), 166.6 (C-7), 117.2 (C-10), 114.8 (C-6) |
| 9 | 169.1 | C | - | |
| 10 | 117.2 | C | - | |
| 1' | 125.3 | C | - | |

(continued)

| Carbon | $\delta_C$ | | $\delta_H$ | Cross peaks ($\delta_C$) in the HMBC spectrum |
|---|---|---|---|---|
| 2' | 117.7 | CH | 7.52 | 149.8 (C-4'), 146.8 (C-3'), 126.7 (C-6'), 116.7 (C-5') |
| 3' | 146.8 | C | - | |
| 4' | 149.8 | C | - | |
| 5' | 116.7 | CH | 6.82 | 146.8 (C-3'), 125.3 (C-1') |
| 6' | 126.7 | CH | 7.31 | 149.8 (C-4'), 117.7 (C-2'), 116.7 (C-5') |

Sugar of the compound (3)

| position | $\delta_C$ | $\delta_H$ |
|---|---|---|
| 1' | 94.2 | 5.15 |
| 2' | 74.7 | 3.55 |
| 3' | 78.4 | 3.60 |
| 4' | 71.3 | 3.45 |
| 5' | 77.8 | 3.55 |
| 6' | 62.4 | 3.98, 3.67 |

Aglycone of the compound (4)

| Carbon | $\delta_C$ | | $\delta_H$ | Cross peaks ($\delta_C$) in the HMBC spectrum |
|---|---|---|---|---|
| 1 | 39.10 | $CH_2$ | 1.61, 0.98 | |
| 2 | 28.79 | $CH_2$ | 1.02, 1.76 | |
| 3 | 90.07 | CH | 3.17 | 4.31 (Glucose-1') |
| 4 | 39.51 | C | - | |
| 5 | 56.32 | CH | 0.76 | |
| 6 | 19.16 | $CH_2$ | 1.41, 1.55 | |
| 7 | 31.93 | $CH_2$ | 1.58, 1.65 | |
| 8 | 40.22 | C | - | |
| 9 | 48.36 | CH | 1.57 | |
| 10 | 37.33 | C | - | |
| 11 | 22.60 | $CH_2$ | 1.87, 1.65 | 122.87 (C-11), 144.87 (C-13) |
| 12 | 122.87 | CH | 5.25 | |
| 13 | 144.87 | C | - | |
| 14 | 42.23 | C | - | |
| 15 | 26.14 | $CH_2$ | 1.92, 1.68 | |
| 16 | 23.16 | $CH_2$ | 1.65, 2.06 | |
| 17 | 47.27 | C | - | |
| 18 | 41.83 | CH | 2.81 | 42.23 (C-14), 47.27 (C-17), 122.87 (C-12) |
| 19 | 46.50 | $CH_2$ | 1.13, 1.71 | 23.33 (C-30), 30.74 (C-20), 32.82 (C-29), 41.83 (C-18) |
| 20 | 30.74 | C | - | |

(continued)

| Carbon | $\delta_C$ | | $\delta_H$ | Cross peaks ($\delta_C$) in the HMBC spectrum |
|---|---|---|---|---|
| 21 | 34.03 | $CH_2$ | 1.21, 2.33 | 30.74 (C-20), 177.78 (C-28) |
| 22 | 33.23 | $CH_2$ | 1.34, 1.46 | |
| 23 | 27.83 | $CH_3$ | 1.04 | 16.81 (C-24), 39.51 (C-4), 56.32 (C-5), 90.07 (C-3) |
| 24 | 16.81 | $CH_3$ | 0.84 | 27.83 (C-23), 39.51 (C-4), 56.32 (C-5), 90.07 (C-3) |
| 25 | 15.28 | $CH_3$ | 0.95 | 37.33 (C-10), 39.10 (C-1), 48.36 (C-9), 56.32 (C-5) |
| 26 | 16.08 | $CH_3$ | 0.79 | 31.93 (C-7), 40.22 (C-8), 42.23 (C-14), 48.36 (C-9) |
| 27 | 25.58 | $CH_3$ | 1.15 | 40.22 (C-8), 42.23 (C-14), 144.87 (C-13) |
| 28 | 177.78 | C | - | 5.45 (Gal-1') |
| 29 | 32.82 | $CH_3$ | 0.90 | 23.33 (C-30), 30.74 (C-20), 34.03 (C-21), 46.50 (C-19) |
| 30 | 23.33 | $CH_3$ | 0.93 | |

Sugars of the compound (4)

| position | $\delta_C$ | $\delta_H$ |
|---|---|---|
| | | |
| Glucose 1' | 105.8 | 4.32 |
| Glucose 2' | 74.7 | 3.19 |
| Glucose 3' | 77.4 | 3.34 |
| Glucose 4' | 70.6 | 3.29 |
| Glucose 5' | 76.8 | 3.24 |
| Glucose 6' | 61.8 | 3.84, 3.68 |
| | | |
| Galactose 1" | 94.5 | 5.45 |
| Galactose 2" | 77.1 | 3.89 |
| Galactose 3" | 71.1 | 3.67 |
| Galactose 4" | 70.0 | 3.37 |
| Galactose 5" | 76.4 (or 78.1) | 3.57 |
| Galactose 6" | 61.3 | 3.80, 3.69 |
| | | |
| Glucose 1'" | 102.7 | 4.80 |
| Glucose 2'" | 71.7 | 3.14 |
| Glucose 3'" | 77.0 | 3.21 |
| Glucose 4'" | 72.8 | 3.37 |
| Glucose 5'" | 77.1 | 3.29 |
| Glucose 6'" | 62.7 | 3.90, 3.62 |

Protocol for the quantitative analysis and titration of the extract A of the Example 1 from *Fadogia ancylantha.*

The title method requires an accurate direct calibration by HPLC. The following experimental conditions were used: Agilent 1100 Series system linked to a G-1312 pump; Rheodyne G-1328A injector (Millipore, Boston, USA); degassifier G 1322 A; loop 20 μl; UV DAD (Diode array detector) G-1315 A detector, for monitoring absorbance, set at

λ 277, 520, 320, 254 nm; Agilent integrator for calculating the areas of the peaks; Thermo Electroporation (150 x 4.6 mm i.d; particle size: 5 p) column; flow: 1 ml/min; injection volume: 20 μl.

The gradient elution was optimized by the following parameters: solvents: 0.1% formic acid in water (solvent A) and methanol (solvent B); gradient 0 to 20 min, 20 to 30% B; 20 to 30 min, 30 to 35% B; 30 to 60 min, 35 to 40% B; 60 to 70 min, 40% B.

Each analysis was carried out in triplicate. The calibration graphics (not herein illustrated) showed a linear relationship between the concentration and the area of the peaks for the compounds (1), (2) and (3).

An amount (15 mg/ml) of the extract A of the Example 1 of *Fadogia ancylantha* was dissolved, for each analysis, in methanol:water 2:8 and subjected to chromatography under the same conditions above illustrated as to the compounds (1), (2) and (3).

The attribution of the peaks was based on the $R_t$ of each compound and confirmed by co-injecting the standards previously isolated.

The results show that the extract A of the Example 1 is composed by 3.0-3.2% of compound (2); 23.2 - 24.0% of compound (1) and 4.8 - 5.2% of compound (3), thus substantially confirming the results illustrated in the following table.

The quantitative analysis and titration of the extract A of the Example 1 were carried out by HPLC-DAD direct calibration; the results are illustrated in the table below:

| | % in extract A (g/100g) |
|---|---|
| compound (1) | 3,1 ± 0,03 |
| compound (2) | 23,7 ± 0,35 |
| compound (3) | 5.0 ± 0,17 |

On the basis of the w/w yield, the compound (4) can be estimated to be 0.0045% in the extract A (g/100g) of the Example 1.

<u>Example 3</u>

A face lifting serum was prepared by mixing the ingredients reported in the table below

| INGREDIENT | % by weight |
|---|---|
| Water | to 100.00 |
| Glycerin | 4.00 |
| Denaturated alcohol | 3.05 |
| Cyclopentasiloxane | 2.00 |
| Dipropylene glycol | 1.00 |
| PEG 90 | 1.00 |
| PEG-40 hydrogenated castor oil | 0.07 |
| Polysorbate 20 | 1.00 |
| Proline | 0.10 |
| Lysine | 0.10 |
| Ceramide 2 | 0.01 |
| Tocopheryl nicotinate | 0.01 |
| Ruscogenin | 0.10 |
| Sodium hyaluronate | 0.10 |
| Xanthan gum | 0.10 |
| Carbomer | 0.50 |

(continued)

| | |
|---|---|
| Urea | 0.20 |
| Ethylhexyl methoxycinnamate | 0.01 |
| Methylparaben | 0.30 |
| Propylparaben | 0.01 |
| Phenoxyethanol | 0.50 |
| DMDM hydantoin | 0.15 |
| Disodium EDTA | 0.05 |
| Sodium hydroxyde | 0.50 |
| Perfume (fragrance) | 0.15 |
| *Fadogia ancylantha* (Extract A of the Example 1) | 0.50 |

Example 4

A deodorizing cream was prepared by mixing the ingredients reported in the table below

| INGREDIENT | % by weight |
|---|---|
| Water | to 100 |
| Aluminium Chlorohydrate | 20.00 |
| Isopropyl Myristate | 20.00 |
| Cetyl Alcohol | 6.00 |
| Glyceryl Stearate | 3.50 |
| PEG - 100 Stearate | 1.50 |
| Laureth - 23 | 1.00 |
| *Fadogia ancylantha* (Extract A of the Example 1) | 0.80 |
| Perfume/Fragrance | 0.50 |
| Farnesol | 0.30 |
| Bisabolol | 0.03 |

Example 5

A deodorizing spray was prepared by mixing the ingredients reported in the table below

| INGREDIENT | % by weight |
|---|---|
| Denaturated Alcohol | 81.489 |
| Water | 15.063 |
| Perfume/Fragrance | 0,500 |
| *Fadogia ancylantha* Extract (Extract A of the Example 1) | 0.600 |
| Triclosan | 0.300 |
| Glycerin | 0.040 |
| *Citrus grandis*/Grapefruit Extract | 0.060 |
| Benzophenone-2 | 0.047 |

(continued)

| INGREDIENT | % by weight |
|---|---|
| Citric Acid | 0.001 |

Example 6

A deterging liquid was prepared by mixing the ingredients reported in the table below

| INGREDIENT | % by weight |
|---|---|
| Water | To 100 |
| PEG-7 Glyceryl Cocoate | 6.0 |
| Sodium Myreth Sulfate | 5.145 |
| Cocamidopropyl Betaine | 3.0 |
| Cocamide DEA | 1.98 |
| Styrene/Acrylates Copolymer | 0.8 |
| Perfume/Fragrance | 0.6 |
| Sodium Polyacrylate | 0.54 |
| *Chamomilla Recutita*/*Chamomilla Recutita* flower extract | 0.5 |
| Ethylhexyl Stearate | 0.315 |
| Imidazolidinyl Urea | 0.3 |
| Glycerin | 0.22 |
| Sodium Methylparaben | 0.2 |
| Propylene Glycol | 0.198 |
| Citric Acid | 0.1 |
| *Triticum vulgare* Hydrolyzed Wheat Protein | 0.1 |
| Disodium EDTA | 0.05 |
| Trideceth-6 | 0.045 |
| *Crataegus monogina*/ *Crataegus monogina* extract | 0.002 |
| *Fadogia ancylantha* (Extract A of the Example 1) | 0.50 |

Example 7

An emollient cream was prepared by mixing the ingredients reported in the table below

| INGREDIENT | % by weight |
|---|---|
| Water | To 100 |
| Cyclopentasiloxane | 8.0 |
| Caprylic/Capric Triglyceride | 5.0 |
| Pentylene Glycol | 5.0 |
| Cetyl Alcohol | 4.0 |
| Steareth-2 | 3.5 |
| Steareth-21 | 3.5 |

(continued)

| INGREDIENT | % by weight |
|---|---|
| Ethyl Hexyl Ethylhexanoate | 2.0 |
| Panthenol | 0.5 |
| Propylene Glycol | 0.5 |
| Zinc Oxide | 0.4 |
| Tocopheryl Acetate | 0.3 |
| Sodium Carboxymethyl Betaglucan | 0.004 |
| Malt Extract | 0.0015 |
| Butylene Glycol | 0.00425 |
| *Oryza* Sativa/Rice Bran Oil | 0.01 |
| Hyaluronic Acid | 0.004 |
| *Citrus Grandis*/Grapefruit Extract | 0.5 |
| Ceramide 3 | 0.005 |
| Ceramide 6-II | 0.0025 |
| Ceramide I | 0.000005 |
| Phytosphingosine | 0.0025 |
| Cholesterol | 0.0025 |
| Sodium Lauroyl Lactylate | 0.05 |
| Carbomer | 0.0015 |
| Xanthan Gum | 0.0015 |
| Disodium EDTA | 0.05 |
| Acrylates/$C_{10-30}$ Alkyl Acrylate Crosspolymer | 0.3 |
| Sodium Hydroxide | 0.2 |
| Retinyl Palmitate | 0.2 |
| BHA | 0.05 |
| *Fadogia Ancylantha* (Extract A of the Example 1) | 0.50 |

Example 8

SAFETY OF USE AND ANTIAGEING EFFECTIVENESS

A study was carried our for 60 days on a group of 20 women volunteers, 35 to 63 years old, showing signs of both ageing and photo-ageing of the face, all women being in good health and having no skin disease, to evaluate the safety and effectiveness of the composition of the present invention.

The volunteers applied on the face the face lifting serum of Example 3 twice a day, each day; no further hydrating nor anti-wrinkle nor pharmaceutical product was applied during the 60 days of the test.

The people were also asked to subject themselves to no dietary regime and, in general, not to change their habits (as to sport, nutrition, etc.) for the whole duration of the test.

Both at the beginning (t0) and at the end (t60) of the test, skin copies of the periocular area of any volunteer were realized. The skin copies were then subjected to profilometry analysis.

Corneometric measurements were carried out on the skin of the face of any volunteer at t0, t30 and t60 so to evaluate skin hydration, measuring the amount of water in the corneous layer; also elastometric measurements were carried out at the same times and conditions so to evaluate skin elasticity, measuring the maximum extensibility and the residual deformation of the skin.

Further, a subjective self-evaluation was asked to the volunteers, carried out at the same times, so to evaluate, hydration(1), softness(1), smoothness(1), lightness(1) and skin rugosity(2) (wrinkles)of the face.

At t60, the volunteers were also asked to express their evaluation of the organoleptic characteristics and of the cosmetic agreeability of the composition of the invention (so-called "psycho-rheological test") so to evaluate the scent(3), colour(3) and consistency(3) of the composition of the invention and whether it had been easily applied(1) and absorbed(1).

(1) on a 0 to 6 scale (0 = poor; 2 = sufficient; 4 = good; 6 = excellent);
(2) on a 0 to 6 scale (0 = absent; 2 = slight; 4 = moderate; 6 = severe)
(3) on a 0 to 6 scale (0 = slightly agreeable; 2 = sufficiently agreeable; 4 = agreeable; 6 = extremely agreeable).

RESULTS

The results illustrated in the table below show that, at t60, the composition of the invention causes a hydration increase equal to 23.12%, determined as the water amount in the corneous layer and an elasticity increase equal to 6.79%, determined by a suction elastometric protocol.

| | corneometric measurements | | | elastometric measurements | | |
|---|---|---|---|---|---|---|
| Volunteer | t0 | t30 | t60 | t0 | t30 | t60 |
| 1 | 60 | 75 | 79 | 0.722 | 0.718 | 0.729 |
| 2 | 61 | 73 | 80 | 0.702 | 0.722 | 0.731 |
| 3 | 52 | 70 | 79 | 0.700 | 0.689 | 0.733 |
| 4 | 51 | 71 | 75 | 0.733 | 0.813 | 0.809 |
| 5 | 75 | 78 | 75 | 0.667 | 0.748 | 0.767 |
| 6 | 79 | 80 | 82 | 0.790 | 0.820 | 0.818 |
| 7 | 47 | 62 | 65 | 0.750 | 0.739 | 0.781 |
| 8 | 62 | 83 | 85 | 0.716 | 0.751 | 0.755 |
| 9 | 61 | 75 | 79 | 0.667 | 0.750 | 0.761 |
| 10 | 62 | 79 | 80 | 0.757 | 0.818 | 0.822 |
| 11 | 48 | 65 | 70 | 0.544 | 0.661 | 0.694 |
| 12 | 54 | 62 | 73 | 0.625 | 0.750 | 0.775 |
| 13 | 68 | 72 | 77 | 0.667 | 0.716 | 0.751 |
| 14 | 68 | 75 | 79 | 0.708 | 0.683 | 0.699 |
| 15 | 62 | 65 | 69 | 0.865 | 0.869 | 0.877 |
| 16 | 48 | 50 | 55 | 0.815 | 0.823 | 0.828 |
| 17 | 61 | 69 | 68 | 0.719 | 0.722 | 0.720 |
| 18 | 44 | 52 | 50 | 0.655 | 0.677 | 0.679 |
| 19 | 60 | 76 | 76 | 0.701 | 0.709 | 0.725 |
| 20 | 62 | 62 | 63 | 0.556 | 0.554 | 0.559 |
| average | 59.25 | 69.70 | 72.95 | 0.703 | 0.737 | 0.751 |

| corneometric measurements % variations | | elastometric measurements % variations | |
|---|---|---|---|
| t0-t30 | t0-t60 | t0-t30 | t0-t60 |
| 17.64% | 23.12% | 4.79% | 6.79% |

The table below illustrates the results of the profilometry analysis of the skin copies denoting a decrease of the average skin rugosity index Rz (which expresses the average depth of wrinkles) equal to 3.39%.

| | Rz (mm) | |
|---|---|---|
| Volunteer | t0 | t60 |
| 1 | 0.126 | 0.120 |
| 2 | 0.148 | 0.131 |
| 3 | 0.173 | 0.162 |
| 4 | 0.121 | 0.119 |
| 5 | 0.155 | 0.157 |
| 6 | 0.096 | 0.099 |
| 7 | 0.157 | 0.150 |
| 8 | 0.121 | 0.115 |
| 9 | 0.136 | 0.128 |
| 10 | 0.144 | 0.132 |
| 11 | 0.118 | 0.108 |
| 12 | 0.167 | 0.166 |
| 13 | 0.122 | 0.125 |
| 14 | 0.130 | 0.127 |
| 15 | 0.123 | 0.121 |
| 16 | 0.145 | 0.140 |
| 17 | 0.118 | 0.114 |
| 18 | 0.166 | 0.167 |
| 19 | 0.137 | 0.131 |
| 20 | 0.173 | 0.170 |
| average | 0.139 | 0.134 |

The two tables below illustrate the results of the subjective self-evaluation and of the psycho-rheological test denoting that any of the measured parameter showed to have been positively affected by the application of the composition of the invention.

No side effects were reported, this showing an excellent tolerability as well.

| | Hydration | | | Softness | | | Smoothness | | |
|---|---|---|---|---|---|---|---|---|---|
| Volunteer | t0 | t30 | t60 | t0 | t30 | t60 | t0 | t30 | t60 |
| 1 | 2 | 3 | 4 | 4 | 4 | 5 | 4 | 4 | 4 |
| 2 | 2 | 3 | 3 | 4 | 4 | 5 | 2 | 3 | 3 |
| 3 | 0 | 4 | 4 | 0 | 4 | 4 | 0 | 4 | 4 |
| 4 | 3 | 4 | 4 | 3 | 4 | 4 | 3 | 4 | 4 |
| 5 | 2 | 4 | 4 | 2 | 3 | 3 | 2 | 3 | 3 |
| 6 | 3 | 4 | 4 | 3 | 4 | 4 | 3 | 4 | 4 |
| 7 | 2 | 6 | 6 | 3 | 6 | 6 | 3 | 4 | 4 |
| 8 | 0 | 1 | 1 | 4 | 4 | 4 | 4 | 4 | 4 |

(continued)

| | Hydration | | | Softness | | | Smoothness | | |
|---|---|---|---|---|---|---|---|---|---|
| Volunteer | t0 | t30 | t60 | t0 | t30 | t60 | t0 | t30 | t60 |
| 9 | 0 | 0 | 2 | 0 | 0 | 2 | 0 | 2 | 3 |
| 10 | 0 | 4 | 4 | 2 | 4 | 4 | 0 | 2 | 3 |
| 11 | 0 | 3 | 4 | 0 | 3 | 4 | 0 | 3 | 4 |
| 12 | 0 | 2 | 3 | 2 | 3 | 3 | 2 | 2 | 3 |
| 13 | 2 | 3 | 4 | 2 | 4 | 4 | 2 | 4 | 4 |
| 14 | 2 | 2 | 2 | 3 | 3 | 4 | 3 | 4 | 4 |
| 15 | 2 | 2 | 3 | 3 | 3 | 3 | 3 | 3 | 4 |
| 16 | 1 | 1 | 1 | 2 | 3 | 3 | 4 | 4 | 4 |
| 17 | 3 | 4 | 4 | 4 | 4 | 4 | 2 | 3 | 3 |
| 18 | 2 | 3 | 3 | 2 | 3 | 3 | 2 | 3 | 3 |
| 19 | 2 | 4 | 4 | 4 | 4 | 4 | 2 | 4 | 4 |
| 20 | 2 | 2 | 2 | 2 | 2 | 2 | 2 | 3 | 3 |
| average | 1.50 | 2.95 | 3.30 | 2.45 | 3.45 | 3.75 | 2.15 | 3.35 | 3.60 |

| Hydration % variation | | Softness % variation | | Smoothness % variation | |
|---|---|---|---|---|---|
| t0-t30 | t0-t60 | t0-t30 | t0-t60 | t0-t30 | t0-t60 |
| 96.67% | 120.00% | 40.82% | 53.06% | 55.81% | 67.44% |

| | Lightness | | | Rugosity | | |
|---|---|---|---|---|---|---|
| Volunteer | t0 | t30 | t60 | t0 | t30 | t60 |
| 1 | 0 | 0 | 0 | 3 | 3 | 3 |
| 2 | 2 | 2 | 2 | 4 | 4 | 3 |
| 3 | 2 | 3 | 3 | 6 | 5 | 5 |
| 4 | 0 | 0 | 0 | 3 | 2 | 2 |
| 5 | 0 | 1 | 1 | 4 | 4 | 3 |
| 6 | 2 | 3 | 3 | 2 | 2 | 2 |
| 7 | 0 | 4 | 4 | 5 | 4 | 4 |
| 8 | 2 | 2 | 2 | 3 | 3 | 3 |
| 9 | 0 | 0 | 0 | 4 | 3 | 3 |
| 10 | 2 | 2 | 2 | 4 | 3 | 3 |
| 11 | 2 | 3 | 4 | 3 | 2 | 2 |
| 12 | 2 | 3 | 3 | 5 | 5 | 5 |
| 13 | 0 | 2 | 2 | 3 | 3 | 2 |
| 14 | 3 | 3 | 3 | 3 | 3 | 3 |
| 15 | 0 | 0 | 0 | 3 | 3 | 3 |
| 16 | 0 | 0 | 0 | 4 | 4 | 4 |
| 17 | 4 | 4 | 4 | 3 | 3 | 3 |

(continued)

| | Lightness | | | Rugosity | | |
|---|---|---|---|---|---|---|
| Volunteer | t0 | t30 | t60 | t0 | t30 | t60 |
| 18 | 2 | 2 | 2 | 5 | 5 | 5 |
| 19 | 2 | 3 | 3 | 4 | 4 | 3 |
| 20 | 0 | 0 | 0 | 6 | 6 | 6 |
| average | 1.25 | 1.85 | 1.90 | 3.85 | 3.55 | 3.35 |
| Lightness % variation | | | Rugosity % variation | | | |
| t0-t30 | t0-t60 | | t0-t30 | | t0-t60 | |
| 48.00% | 52.00% | | -7.79% | | -12.99% | |

| | Smell | Colour | Consistency | Application ease | Absorbing ease |
|---|---|---|---|---|---|
| Volunteer | t60 | t60 | t60 | t60 | t60 |
| 1 | 4 | 4 | 2 | 4 | 4 |
| 2 | 4 | 4 | 4 | 4 | 4 |
| 3 | 4 | 4 | 4 | 4 | 4 |
| 4 | 4 | 4 | 4 | 4 | 4 |
| 5 | 6 | 4 | 4 | 4 | 6 |
| 6 | 4 | 4 | 2 | 4 | 2 |
| 7 | 4 | 4 | 4 | 6 | 6 |
| 8 | 2 | 2 | 4 | 4 | 3 |
| 9 | 4 | 4 | 4 | 4 | 2 |
| 10 | 4 | 4 | 4 | 4 | 6 |
| 11 | 4 | 4 | 3 | 4 | 3 |
| 12 | 4 | 4 | 4 | 4 | 4 |
| 13 | 4 | 4 | 6 | 4 | 6 |
| 14 | 4 | 4 | 2 | 4 | 2 |
| 15 | 4 | 4 | 2 | 4 | 2 |
| 16 | 4 | 4 | 4 | 4 | 4 |
| 17 | 4 | 4 | 4 | 4 | 4 |
| 18 | 6 | 4 | 4 | 4 | 6 |
| 19 | 4 | 4 | 2 | 4 | 2 |
| 20 | 4 | 4 | 4 | 4 | 4 |
| average | 4.1 | 3.9 | 3.6 | 4.1 | 3.9 |

In view of the foregoing, it can be therefore noted that the composition of the invention has shown an excellent cosmetic effectiveness in adjuvating the treatment of skin showing signs of both chrono- and ageing photo- ageing, inducing an increase of both hydration and elasticity and a decrease of the average depth of wrinkles.

MATERIALS AND METHODS

Corneometry: the water amount present in the face corneous layer was determined for each volunteer using the Corneometer CM 825 (Courage + Khazaka). The average values and percentage variations were measured at t0, t30 and t60.
The standard corneometric values are higher than 60.
Elastometry: the maximum extensibility (R0) and the residual deformation (R1) were measured on the face of each volunteer using the Cutometer Sem 474 (Courage + Khazaka). The average values and percentage variations of the final elasticity (Rz) were measured by the following formula: R2=(R0-R1/R0) wherein R0 is the maximum value of the extension and R1 is the cutaneous shrinkage. As a standard, the values of Rz are between 0 and 1, where 0 corresponds to the minimum elasticity and R1 corresponds to the maximum value thereof.
Skin copies: the siliconic resin Silflo (Flexico) was used to make skin copies of the periocular area of the face of each volunteer so to evaluate skin rugosity.
Profilometry analysis of skin copies: the average index of skin rugosity (Rz), expressed as the average depth of wrinkles measured in millimetres, was measured for each of the skin copies. Rz varies, as a standard, between 0.08 and 0.20 mm for *"normal skin".*
The average values and percentage variations of the scores given to the parameters taken into consideration were determined at t0, t30 and t60, according to the respective scales of evaluation, during the self-evaluation and the psycho-rheological tests.
All the data collected were subjected to statistical analysis.

<u>Example 9</u>

ANTIMICROBIAL ACTIVITY

The antimicrobial activity of the methanol extract A of the Example 1 of *Fadogia ancylantha,* obtained as above illustrated in Example 1, was evaluated applying the method of microdilutions in broth as disclosed by Koneman E. W., 1995, "Testo atlante di Microbiologia Diagnostica", 2nd ed. A. Delfino, Roma, pages 550-605 and Camporese A., 1997, "L'aromatogramma: metodi, corretto utilizzo, prospettive di ricerca", Rivista Italiana EPPOS 21, 4 and in Ismaili, H. et al. "In vivo anti-inflammatory and in vitro antioxidant activities of two extracts of Thymus satureioides leaves". J. of Ethnoph. 2004, 91, 31-36.
The following CULTI-LOOPS (OXOID), prepared so to have a concentration of $10^5$ microorganisms/ml, were used:

*Staphylococcus aureus* ATCC 6538; *Pseudomonas aeruginosa* ATCC 9027 and *Candida albicans* ATCC 10231. The concentrations of the extract A of the Example 1 subjected to the analysis were of 20 mg/ml to 0,019 mg/ml.

A blank control was also prepared using 100 to 1.5625 μg/ml of DMSO so to show the absence of any antimicrobial activity of DMSO which was used to solubilize the extract A of the Example 1.

Results

Test on the extract A of the Example 1 of *Fadogia ancylantha* on *Staphylococcus aureus* ATCC 6538: the MIC observed on scalar concentrations of the extract A of the Example 1 of 20mg/ml to 0.019 mg/ml was 10 mg/ml > MIC > 5 mg/ml.
Test on the extract A of the Example 1 of *Fadogia ancylantha* on *Candida albicans* ATCC 10231: the MIC observed on scalar concentrations of the extract A of the Example 1 of 20mg/ml to 0.019 mg/ml was 20 mg/ml > MIC > 10 mg/ml.
Test on the extract A of the Example 1 of *Fadogia ancylantha* on *Pseudomonas aeruginosa* ATCC 9027: the MIC observed on scalar concentrations of the extract A of the Example 1 of 20 mg/ml to 0.019 mg/ml was higher than 20 mg/ml.

ANTIOXIDANT ACTIVITY

The antioxidant activity of the dry extract A, obtained as above illustrated in Example 1, of *Fadogia ancylantha* was determined using the 2,2 diphenyl-1-picrilhydrazilic stable radical (DPPH°) and α-tocopherol as a control, according to the protocol disclosed by Aquino R. et al. in "Phenolic constituents and antioxidant activity of an extract of Anthurium versicolor leaves", J. of Natural products 64, 1019-1023.

The decrease of absorbance measured according to the above protocol is directly correlated with the free-radical scavenger ability of the antioxidant under test.

The DPPH° percentage remaining at the steady state (%$DPPH°_{RIM}$) is calculated by applying the following formula

$$\% \ DPPH^{\circ}_{RIM} = (DPPH^{\circ}) \ x \ T_1/(DPPH^{\circ}) \ x \ T_o \ x \ 100$$

where $T_1$ is the time to reach the steady state.

The amount of the antioxidant needed to reduce the radical initial concentration to a half ($SC_{50}$, expressed as µg of the extract/ml) was calculated from the percentage of $DPPH°_{RIM}$ with respect to the antioxidant concentrations used.

All tests were carried out in triplicate and repeated for three times.

Besides, the analysis of total phenol in the dry extract A of the Example 1 of *Fadogia ancylantha,* obtained as above illustrated in Example 1, was carried out following the colorimetric method of Folin-Ciocalteau (phosphour-molibden-tungsten reactant, Aquino, R. et al. "An extract of Tagetes lucida and its phenolic constituents as antioxidant". J. of Nat. prod. 65, 1773-76).

The total phenol was expressed as µg of 2',4,4 trihydroxydihydrochalcone [compound (3)] per mg of the extract

A of the Example 1, knowing from preliminary tests that such compound resulted to be one of the main phenols comprised in the extract A of the Example 1.

Since natural polyphenols are known as substances showing an antioxidant/free-radical scavenger activity, this analysis is useful to point out possible correlations between polyphenol concentration and the radical scavenger activity of a complex plant extract.

The table below shows the coherence between the results obtained for each polyphenol by HPLC-DAD and the ones of the analysis according to Folin-Ciocalteau

| | % in extract A (g/100g) |
|---|---|
| compound (1) | 3,1 ± 0,03 |
| compound (2) | 23,7 ± 0,35 |
| compound (3) | 5.0 ± 0,17 |
| total flavonoid (HPLC) | 31.8 |
| total polyphenol (Folin-Ciocalteau) | 33.2 |

The table below compares the results obtained by the analysis according to Folin-Ciocalteau with the ones of the DPPH° test

| | Total polyphenols[a] µg/ml of the extract[b] | DPPH test $SC_{50}$ µg/ml of the extract A µg/ml of phenols | |
|---|---|---|---|
| *F. ancylantha* (extract A of Example 1) | 331.79 (327.244-336.336)[c] | 77.871 (77.024-78.718)[c] | |
| α-tocopherol[d] | | | 10.1 (8.8-11.4)[c] |
| a = mean ± S.D. of three calculations made applying the Folin-Ciocalteau method; b = chalcone equivalents; c = tolerated range 95%; d = control. | | | |

DEODORIZING EFFECTIVENESS (CREAM)

An 8 hours "short-test" was carried out on a group of 20 volunteers (not belonging to the other groups used for the above illustrated tests), both men and women, 18 to 55 years old, all in good health and having no skin disease, to evaluate the deodorizing effectiveness of the deodorizing cream of Example 4.

At the beginning of the test, the armpits were washed using a standard detergent and soon after, the deodorizing cream of Example 4 was applied on the right armpit only, the left one having been used as a control.

After 4 hours ($t4^{th}$) and 8 hours ($t8^{th}$), the volunteers were subjected to both the objective and subjective odour

analysis of the armpits (so called "sniff-test", according a predefinite scale 0 to 6 where 0=absent, 2=slight, 4=moderate; 6=persistent) and calculating the average values and percentage changes of the values measured for the body odour at both $t4^{th}$ and $t8^{th}$).

After 8 hours from the application of the composition of the invention, samples of the bacterial flora of the armpits were taken from each volunteer so to evaluate, *in vitro,* the antimicrobial activity of the composition of the invention.

The samples of the bacterial flora were subjected to microbial counting according to the analytical protocol of Farmacopea Ufficiale Italiana, X edition, 1998.

Both the objective and subjective evaluations of the body odour ("sniff-test") did report a slight body odour for the treated (right) armpit (average values: t4h=0.3; t8h=2.8 for the objective test - t4h=0.3; t8h=1.7 for the subjective test) whereas the non-treated (left) armpit resulted to smell moderately (average values: t4h=0.4 t8h=3.9 for the objective test - t4h=0.4; t8h=3.8 for the subjective test)

The results of the microbiological analysis showed a good microbacterial activity of the composition of the invention, suitable to limit the formation of the body odour, even after 8 hours. ($14.3 \times 10^5$ as an average total bacterial counting of the treated armpit against $6.2 \times 10^3$ of the non treated one).

DEODORIZING EFFECTIVENESS (SPRAY)

The 8 hours "short-test" above illustrated was repeated to evaluate the deodorizing effectiveness of the deodorizing spray of Example 5, following the same protocol and conditions and yet replacing the people subjected to the previous test.

Both the objective and subjective evaluations of the body odour ("sniff-test") did report at the end of the test a slight body odour for the treated (right) armpit (average values: t4h=0.5; t8h=3.2 for the objective test - t4h=0.6; t8h=3.2 for the subjective test) whereas the non-treated (left) armpit resulted to smell moderately (average values: t4h=0.8 t8h=4.2 for the objective test - t4h=0.8; t8h=4.5 for the subjective test)

The results of the microbiological analysis showed a good microbacterial activity of the composition of the invention, suitable to limit the formation of the body odour, even after 8 hours. ($12.8 \times 10^5$ as an average total bacterial counting of the treated armpit against $4.2 \times 10^4$ of the non treated one).

PRESERVING EFFECTIVENESS (DETERGING LIQUID)

The microbiologic stability of the deterging liquid of Example 6 was evaluated by the so-called *"Challenge test"* which comprises inoculating the composition under test with some microorganisms and controlling their survival in time. The results obtained allow to establish reasonably the preservation ability of the composition under test.

The table herebelow shows the four different groups of microorganisms inoculated during the test, together with their respective compositions, and the final concentrations thereof, expressed as CFU (Colony Forming Units) per gram or milliliter measured in the inoculated sample.

| Microbial groups | C.F.U. | Microorganisms |
|---|---|---|
| Gram + bacteria | $6,2 \times 10^8$ | *Staphylococcus aureus* ATCC 6538 *Staphylococcus epidermidis* ATCC 12228 |
| Gram - bacteria | $3,3 \times 10^8$ | *Escherichia coli* ATCC 8739 *Pseudomonas aeruginosa* ATCC 9027 *Klebsiella pneumoniae* ATCC 10031 *Pseudomonas cepacea* ATCC 25609 |
| Filamentous fungi | $3,6 \times 10^7$ | *Aspergillus niger* ATCC 16404 |
| Yeasts | $5,6 \times 10^7$ | *Candida albicans* ATCC 10231 *Saccharomyces cerevisiae* ATCC 9763 |

Controls of the microbial survival were carried out on the inoculated samples by countings in solid groups after 1, 7 and 28 days. The control carried out after one day is an index of the speed of the preserving system whereas the control after 7 days represents the degree of the risk that the composition under test be contaminated. The final control, after 28 days, besides to being a confirmation of the data previously obtained, allows to determine possible formations of resistant strains.

Before the composition was inoculated, mesophilic aerobic microorganisms amounted to less than 10 CFU/g.

The table below shows the values of the microbial survival expressed as CFU.

| SURVIVAL | GRAM+ | GRAM- | FUNGI | YEASTS |
|---|---|---|---|---|
| After 1 day | <10 | <10 | <10 | $2.4 \times 10^3$ |

(continued)

| SURVIVAL | GRAM+ | GRAM- | FUNGI | YEASTS |
|---|---|---|---|---|
| After 7 days | <10 | <10 | <10 | <10 |
| After 28 days | <10 | <10 | <10 | <10 |

PRESERVING EFFECTIVENESS (EMOLLIENT CREAM)

The microbiologic stability of the emollient cream of Example 7 was evaluated by applying the same protocol and conditions above illustrated for testing the microbiologic stability of the detergent liquid of Example 6 and yet replacing the people subjected to the previous test.

The table herebelow shows the four different groups of microorganisms inoculated during the test, together with their respective compositions, and the final concentrations thereof, expressed as CFU (Colony Forming Units) per gram or milliliter measured in the inoculated sample.

| Microbial groups | C.F.U. | Microorganisms |
|---|---|---|
| Gram + bacteria | $6.2 \times 10^7$ | *Staphylococcus aureus* ATCC 6538 *Staphylococcus epidermidis* ATCC 12228 |
| Gram - bacteria | $7.7 \times 10^7$ | *Escherichia coli* ATCC 8739 *Pseudomonas aeruginosa* ATCC 9027 *Enterobacter cloacae* ATCC 13047 *Pseudomonas putida* |
| Filamentous fungi | $3.8 \times 10^5$ | *Aspergillus niger* ATCC 16404 *Penicillium notatum* ATCC 9179 |
| Yeasts | $1.3 \times 10^7$ | *Candida albicans* ATCC 10231 *Saccharomyces cerevisiae* ATCC 9763 |

Before the composition was inoculated, mesophilic aerobic microorganisms amounted to less than 10 CFU/g.

The table below shows the values of the microbial survival expressed as CFU.

| SURVIVAL | GRAM+ | GRAM- | FUNGI | YEASTS |
|---|---|---|---|---|
| After 1 day | $10^2$ | $10^2$ | $5.7 \times 10^3$ | $6.5 \times 10^3$ |
| After 7 days | <10 | <10 | $4.3 \times 10^2$ | <10 |
| After 28 days | <10 | <10 | <10 | <10 |

## Claims

**1.** A topical cosmetic and/or dermopharmaceutical composition comprising at least one effective amount of a plant ingredient or plant extract from *Fadogia Ancylantha* and/or parts thereof, with the proviso that the quantitative data add up to 100% by weight with

a) at least one cosmetically and/or dermopharmaceutically acceptable preservative-antioxidant, and
b) water or at least one cosmetically and/or dermopharmaceutically acceptable excipient-emulsifier-liophilic substance, and optionally
c) at least one cosmetically and/or dermopharmaceutically acceptable auxiliary.

**2.** Composition according to claim 1, wherein the at least one plant ingredient or plant extract is present in an amount from about 0.1 to about 5.0% by weight of the total composition.

**3.** Composition according to claim 1 or 2, wherein the at least one plant ingredient or plant extract is present in an amount from about 0.3 to about 3.0% by weight of the total composition.

**4.** Composition according to any of the previous claims, wherein the at least one plant ingredient or plant extract is present in an amount from about 0.5 to about 1.0% by weight of the total composition.

**5.** Composition according to any of the previous claims, wherein the plant extract and/or ingredient comprises at least

one of the following compounds:

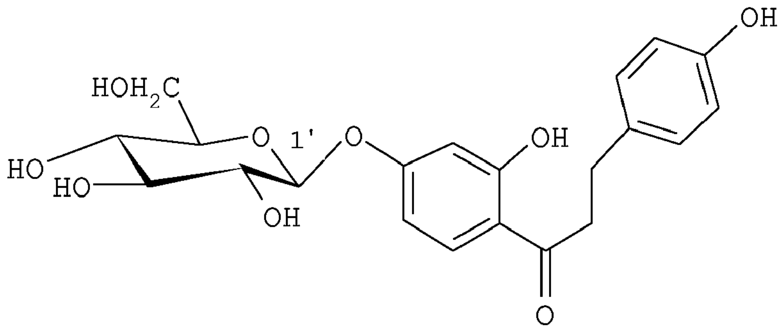

(1)

4'-β-O-D-glucopyranosyl-4,2',4'-trihydroxydihydrochalcone;

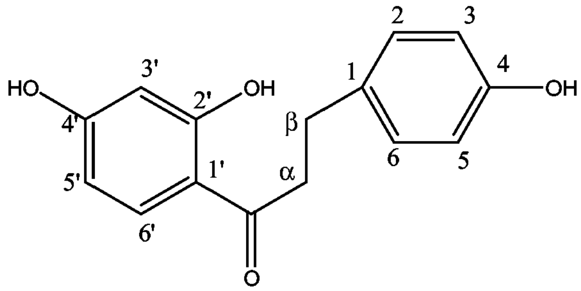

(2)

4,2',4'-trihydroxydihydrochalcone;

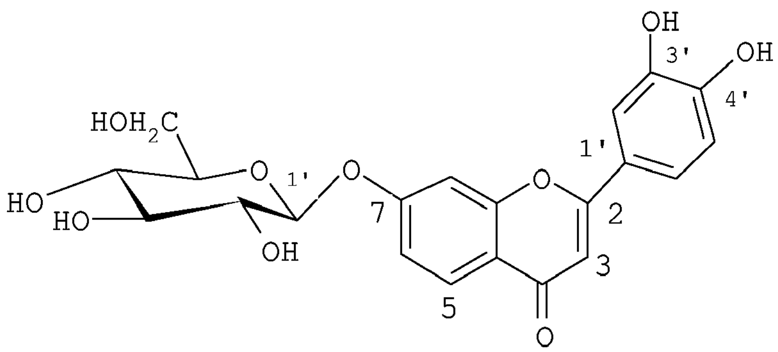

(3)

7-O-β-D-glucopyranosyl-7,3',4'-trihydroxyflavone;

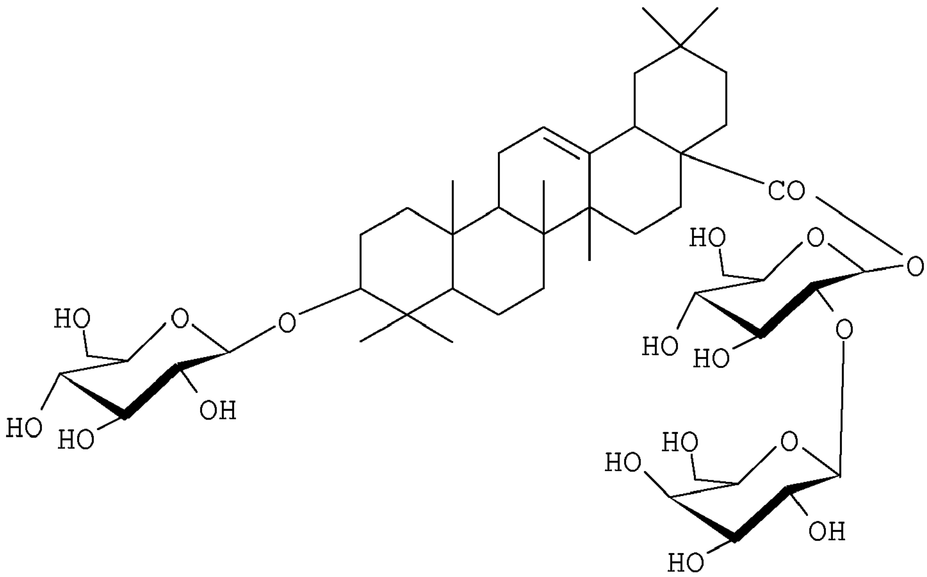

(4)

3-O-β-D-glucopyranosyl-olean-12-en-28-O-[β-D-glucopyranosyl (1→2) galactopyranoside].

6. Composition according to any of the previous claims, wherein the extract is obtainable by

a) extracting the plant *Fadogia Ancylantha* or parts of this plant with a solvent selected from the group consisting of water, a $C_1$-$C_4$ alcohol and mixtures thereof so that a solution of the extract in the solvent is obtained, and
b) removing the solvent from this solution, so that the extract is obtained.

7. Composition according to any of the previous claims, wherein the composition is in a product form selected from the group consisting of aerosol, cream, emulsion, solid, liquid, dispersion, foam, gel, lotion, micro-system, modulated-lypophilic system, mousse, nano-system, ointment, powder, patch, pomade, serum, solution, pump spray, stick, towelette, and combinations thereof.

8. Composition according to any of the previous claims, wherein the at least one cosmetically and/or dermopharmaceutically acceptable auxiliary includes one or more ingredients selected from the group consisting of additives-formulation auxiliaries-solvents; surfactants-solubilizers; natural substances; polymers-rheological modifiers; silicone derivatives; pigments-inorganic additives; humectants; functional substances; functional additives.

9. A compound having the following structural formula:

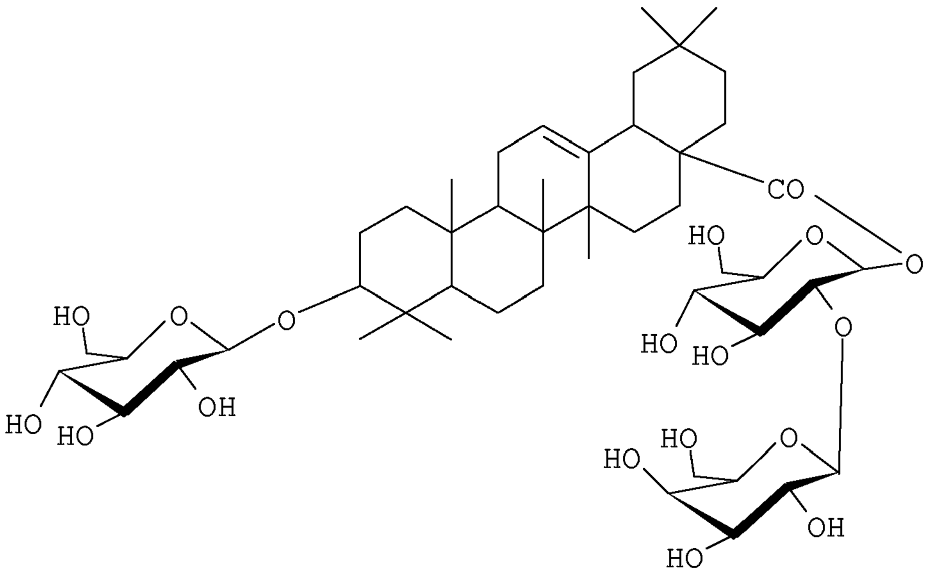

(4)

3-O-β-D-glucopyranosyl-olean-12-en-28-O-[β-D-glucopyranosyl (1→2) galactopyranoside].

**10.** Compound according to the previous claim obtainable by

a) extracting the plant *Fadogia Ancylantha* or parts of this plant with a solvent selected from the group consisting of water, a $C_1$-$C_4$ alcohol and mixtures thereof so that a solution of the extract in the solvent is obtained;
b) removing the solvent from this solution, so that the extract is obtained;
c) freeze-drying and drying the extract;
d) separating the desired compound from the dry freeze-dried extract.

**11.** Use of an extract and/or ingredient of *Fadogia ancylantha* and/or of the composition and/or the compound according to any of the previous claims for the cosmetic and/or dermopharmaceutical treatment of the human body.

**12.** Use according to the previous claim, whereby the cosmetic and/or dermopharmaceutical treatment comprises simultaneously an anti-ageing, anti-wrinkle, anti-bacterial, antioxidant and anti-free-radicals effect on aged, photo-aged, stressed or tired skin.

**13.** A method of improving at least one sign of ageing in skin, comprising topically applying to the skin the composition or compound according to any of claims 1 to 10, in a cosmetically or dermopharmaceutically effective amount sufficient to improve at least one sign of ageing in skin.

**14.** The method according to the previous claim, where the at least one sign of ageing is selected from the group consisting of lines, fine lines, wrinkles, crow's feet, dark eye circles, blemishes, age spots, and stretch marks.

**15.** The method according to claim 13 or 14, wherein the at least one sign of ageing results from at least one of free radical damage, environmental agents, pollutants, diet, chronological ageing, premature ageing, hormonal ageing, and photo-ageing.

**16.** Use of a plant ingredient or plant extract from *Fadogia ancylantha* as active agent having simultaneously protective and repairing properties, for the preparation of a composition according to any of claims 1 to 8, for the cosmetic and/or dermopharmaceutical treatment of the human body.

European Patent Office

# EUROPEAN SEARCH REPORT

Application Number
EP 05 11 1134

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DARVISH: "Makoni herbal tea" HOLISTIQUE LETTER, vol. 1, no. 3, April 2004 (2004-04), pages 1-8, XP002382123 * See page 4, esp. the reference to healthy skin * ----- | 1-16 | INV. A61K36/74 A61P17/00 |
| A | VAN PUYVELDE: "Wheat rootlet growth inhibition test of Rwandese medicinal plants: Active principles of Tetradenia riparia and Diplolophium africanum" JOURNAL OF ETHNOPHARMACOLOGY, vol. 24, 1988, pages 233-246, XP002382124 * See pages 236 and 243 ((leaf, stem / cough, ascaris) * ----- | 1-16 | |

TECHNICAL FIELDS SEARCHED (IPC)
A61K
A61P

The present search report has been drawn up for all claims

| Place of search | Date of completion of the search | Examiner |
|---|---|---|
| Berlin | 24 May 2006 | Korsner, S-E |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..............................................
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SANON S. et al.** Ethnobotanical survey and in vitro antispasmodial activity of plants used in traditional medicine in Burkina Faso. *J. of Ethnopharmacology,* 2003, vol. 86 (2-3), 143-7
- **KONEMAN E. W.** Testo atlante di Microbiologia Diagnostica. 1995, 550-605
- **CAMPORESE A.** L'aromatogramma: metodi, corretto utilizzo, prospettive di ricerca. *Rivista Italiana EPPOS,* 1997, vol. 21, 4
- **ISMAILI, H. et al.** In vivo anti-inflammatory and in vitro antioxidant activities of two extracts of Thymus satureioides leaves. *J. of Ethnoph.,* 2004, vol. 91, 31-36
- **AQUINO R. et al.** Phenolic constituents and antioxidant activity of an extract of Anthurium versicolor leaves. *J. of Natural products,* vol. 64, 1019-1023
- **AQUINO, R. et al.** An extract of Tagetes lucida and its phenolic constituents as antioxidant. *J. of Nat. prod.,* vol. 65, 1773-76